**Europäisches Patentamt**

**European Patent Office**

**Office européen des brevets**

(19)

(11) Publication number: **0 049 559**
**A2**

(12) # EUROPEAN PATENT APPLICATION

(21) Application number: **81302409.8**

(22) Date of filing: **01.06.81**

(51) Int. Cl.³: **A 61 K 6/08**
**C 08 L 33/00**

(30) Priority: **29.09.80 US 191939**

(43) Date of publication of application:
**14.04.82 Bulletin 82/15**

(84) Designated Contracting States:
**AT BE CH DE FR GB IT LI NL SE**

(71) Applicant: **SYBRON CORPORATION**
**1100 Midtown Tower**
**Rochester, NY 14604(US)**

(72) Inventor: **Waller, Duncan E.**
**9670 Woodland Court**
**Ypsilanti Michigan 48197(US)**

(74) Representative: **Oliver, Roy Edward et al,**
**POLLAK, MERCER & TENCH High Holborn House 52-54**
**High Holborn**
**London WC1V 6RY(GB)**

(54) **Dental restorative compositions.**

(57) Two-paste dental restorative compositions cured by a free-radical-generating peroxide/amine redox combination contain either or both of ethoxylated bisphenol A dimethacrylate and propoxylated bisphenol A dimethacrylate, optionally blended with bisphenol A/glycidyl methacrylate adduct, as a monomeric matrix and one or more inorganic fillers selected from strontium aluminium borosilicate, barium aluminium silicate, barium aluminium borosilicate and mixtures thereof.

EP 0 049 559 A2

- 1 -

DENTAL RESTORATIVE COMPOSITIONS

DESCRIPTION

This invention relates to dental restorative materials. Since dentistry was first practised, dentists and patients alike have sought the ideal restorative material and the search still continues. The literature shows that many innovations have been published and patented, some of much greater significance than others.

However, within the past decade, composite materials based upon vitreous filled combinations of acrylic functional monomers, pioneered by R.L. Bowen and described in U.S. Patent Specification 3,006,112, have gained both professional and public acceptance and have proliferated in ever-increasing variations. Within this proliferation, certain desirable and virtually essential characteristics have become generally recognised and established, namely, (i) two-paste equi-ratio mixing, (ii) pre-shaded compositions for easy placement preparation, (iii) a sufficient radiopaque filler content to enable certain radiographic detection to be effected, (iv) adequate shelf stability, (v) physical properties approaching or even exceeding those of human tooth enamel and (vi) relative ease of finishing.

One characteristic of restorative compositions, which is highly desirable when used for anterior restorations and is virtually essential when used for posterior restorations, especially in occlusal load-bearing locations, is sufficient abrasion-resistance to ensure maintenance of reasonable anatomical form for a duration of several years. Compared with the abrasion resistance of the better amalgam restorations, this characteristic has generally remained elusive during more than 15 years of development of dental composites. While masticatory forces normally serve to burnish and polish the occlusal surface of amalgam restorations, in the case of composite restorations, a two-phase erosion pattern is observable, whereby progressive erosion of the polymeric matrix results in eventual dislodgement of filler particles and this exposes new areas of the matrix to the erosion process.

In order to retard this erosion process significantly, changes in the characteristics of both the polymeric and vitreous phases appear to be necessary. The polymeric phase should remain relatively rigid, yet be less brittle and therefore tougher. This predicates monomer molecules of fairly rigid structure, low polymerization shrinkage and optimum crosslinking density. The filler particles should be of optimum particle size distribution for maximum volume fraction packing, have adequate hardness yet not be brittle and should closely match the refractive index of both tooth enamel and the polymeric matrix to ensure reasonable aesthetic appeal.

Traditionally, the vast majority of composite dental restoratives have embodied a monomeric matrix consisting of BisGMA resin, i.e. bisphenol A/glycidyl methacrylate adduct resin, diluted to a suitable viscosity with one or more diacrylate functional monomers. Most of these diluent monomers are of relatively low molecular weight, resulting in high polymerization shrinkage, high crosslinking density and brittleness due to residual matrix stress and high modulus, eventually forming micro-cracks. Following later work, R.L. Bowen reported the synthesis of a liquid eutectic monomer blend based on isomeric phthaloyl dimethacrylates, but as yet this has not achieved commercial utility due to several inherent disadvantages.

There is thus a continuing need for improved abrasion-resistant dental composites which overcome the above-noted disadvantages and this invention seeks to provide such improved dental composites, advantageously in the form of two-paste systems.

The use of crystalline monomers, wholly or as diluents for BisGMA resin, has received little attention, particularly the use of crystalline monomers of closely-related structure and molecular weight. It has surprisingly been discovered that both ethoxylated and propoxylated bisphenol A dimethacrylates can be obtained in a very pure crystalline state, by reacting bisphenol A with either ethylene or propylene carbonate as appropriate, followed by esterification, though slight traces of impurities cause liquification at ambient temperature due to their low melting point. When optionally blended in widely variable proportions with BisGMA resin at slightly elevated temperature, dissolution occurs and the resulting solution remains stable with respect to freedom from crystal deposition, even after prolonged refrigeration. Furthermore, following easy and rapid ambient temperature copolymerization, a tough moderately-crosslinked fracture-resistant polymer results, with low attendant polymerization shrinkage.

According to one aspect of this invention, therefore, a dental restorative composition is provided, which is curable by means of a free-radical-generating peroxide/amine redox combination and comprising an acrylic functional monomer and at least one filler material, which is characterized in that the monomer comprises at least one alkoxylated bisphenol A dimethacrylate and that the filler material is selected from strontium aluminium borosilicate, barium aluminium silicate, barium aluminium borosilicate and mixtures thereof.

The compositions of the present invention may advantageously include either or both of ethoxylated and propoxylated bisphenol A dimethacrylates, alone or in combination with BisGMA resin, but they do not comprise BisGMA resin alone. A desired concentration of ethoxylated or propoxylated bisphenol A dimethacrylate, alone or in combination, is in the range from 15 to 30 weight percent. The BisGMA is preferably present, when used, in concentrations of up to 15 weight percent.

If one of the above monomers or a blend of the two monomers of suitable viscosity is then highly loaded with fillers of optimal particle size distribution, chosen from the aforementioned particulate materials of suitable modulus and refractive index, together with the necessary catalyst and accelerator components plus optional pigments and stabilizers, a two-paste system can be formulated which, when adequately polymerized, exhibits abrasion resistance superior to that of all known commercial systems presently in use. A suitable size distribution for the filler particles is from 0.02 to 30 microns, with approximately 50 weight percent of the particles preferably being in the 1.0 to 10.0 micron size range. The filler material may typically be present as a single material or as a mixture of materials in a total concentration of 70 to 85 weight percent. The two pastes are normally always spatulated together, to initiate the redox reaction, which results in polymerization and setting of the material.

Two typical examples of such two-paste systems are as follows:

EXAMPLE 1

CATALYST PASTE

ethoxylated bisphenol A dimethacrylate monomer BisGMA resin

benzoyl peroxide catalyst

UV absorber

submicron silicon dioxide

strontium aluminium borosilicate filler

BASE PASTE

ethoxylated bisphenol A dimethacrylate monomer BisGMA resin

tertiary amine accelerator

submicron silicon dioxide

barium aluminium borosilicate

UV absorber

pigments

EXAMPLE 2

CATALYST PASTE

ethoxylated bisphenol A dimethacrylate monomer benzoyl peroxide catalyst

UV absorber

submicron silicon dioxide

strontium aluminium borosilicate filler

BASE PASTE

propoxylated bisphenol A dimethacrylate monomer tertiary amine accelerator

UV absorber

pigments

submicron silica

barium aluminium silicate filler

A variety of synthetic vitreous materials based on melts containing substantial amounts of alumina and silica, plus a sufficient quantity of at least one metallic oxide designed to impart radiopacity, are preferred. Fused alumina possesses the necessary abrasion resistance, but is of unsuitable refractive index and is essentially nonradiopaque. Many porcelain tooth glazes also possess the necessary abrasion resistance and refractive index, but are essentially non-radiopaque. A combination of submicron silica and polymeric filler yields a composite which can be polished almost as smooth as amalgam, but has very poor abrasion resistance, though it is used commercially in at least two anterior restorative formulations. The submicron silica may typically be present in a concentration of up to 10 weight percent. It has been discovered that radiopaque fillers of vitreous materials containing substantial amounts of barium or strontium oxide fused with appropriate quantities of alumina and silica to yield stable leach-resistant glasses are particularly suitable for use with the above-described monomers.

The following are further Examples of various compositions falling within the scope of the present invention. The percentages listed in the Examples are by weight:

EXAMPLE 3

A two-paste filled composite restorative material based on the standard peroxide/amine curing system and having the following final mix composition was prepared by mixing and reacting the ingredients by spatulation:

| | |
|---|---|
| ethoxylated bisphenol A dimethacrylate | 10.475 |
| bisphenol A/glycidyl methacrylate adduct | 10.475 |
| benzoyl peroxide | 0.23 |
| tertiary amine | 0.20 |
| UV absorber | 0.10 |
| 2,6-di-tertiary-butyl-para-cresol | 0.02 |
| strontium aluminium borosilicate | 78.50 |
| | 100.00 |

An abrasion rate of 1.46 microlitres/hour was measured for this material seven days from the time of mixing.

EXAMPLE 4

A similar two-paste composite material utilizing a peroxide/amine curing system and having the following final mix composition was prepared in a manner similar to that set forth in Example 3.

| | |
|---|---|
| bisphenol A/glycidyl methacrylate adduct | 16.60 |
| triethylene glycol dimethacrylate (inhibited) | 2.27 |
| butylene glycol dimethacrylate (inhibited) | 2.09 |
| tertiary amine | 0.25 |
| UV absorber | 0.20 |
| benzoyl peroxide | 0.09 |
| strontium aluminium borosilicate | 78.50 |
| | 100.00 |

This composition gave an increased abrasion rate of 1.81 microlitres/hour, measured seven days from the time of mixing, compared with the materials of Example 3.

EXAMPLE 5

Another peroxide/amine cured two-paste composite material was prepared, having the following final mix composition, in a manner similar to that set forth in Example 3:

| propoxylated bisphenol A dimethacrylate | 21.50 |
|---|---|
| benzoyl peroxide | 0.25 |
| tertiary amine | 0.25 |
| UV absorber | 0.10 |
| 2,6-di-tertiary-butyl-para-cresol | 0.02 |
| FD&C-approved pigment | 0.75 |
| submicron silicon dioxide | 1.50 |
| strontium aluminium borosilicate | 75.63 |
| | 100.00 |

This material gave an abrasion rate of 1.55 microlitres/hour, measured seven days from the time of mixing, which was not significantly different from the material of Example 3.

EXAMPLE 6

A further peroxide/amine cured two-paste composite material, having the following final mix composition, was prepared in a manner similar to that set forth in Example 3.

| ethoxylated bisphenol A dimethacrylate | 18.69 |
|---|---|
| benzoyl peroxide | 0.30 |
| tertiary amine | 0.28 |
| UV absorber | 0.09 |
| 2,6-di-tertiary-butyl-para-cresol | 0.02 |
| submicron silicon dioxide | 1.39 |
| barium aluminium silicate | 41.10 |
| strontium aluminium borosilicate | 38.13 |
| | 100.00 |

This composition gave a significantly reduced abrasion rate of 1.12 microlitres/hour, measured seven days from the time of mixing, when compared with the material of Example 3.

EXAMPLE 7

An additional peroxide/amine cured two-paste composite material, having the following final mix composition, was prepared in a manner similar to that set forth in Example 3:

| | |
|---|---:|
| ethoxylated bisphenol A dimethacrylate | 21.50 |
| benzoyl peroxide | 0.30 |
| tertiary amine | 0.30 |
| UV absorber | 0.10 |
| 2,6-di-tertiary-butyl-para-cresol | 0.02 |
| submicron silicon dioxide | 1.50 |
| strontium aluminium borosilicate | 76.28 |
| | 100.00 |

This material gave an abrasion rate of 1.31 microlitres/hour, measured seven days from the time of mixing, which was not significantly different from the material of Example 3.

EXAMPLE 8

Another peroxide/amine cured two-paste composite material, having the following final mix composition, was prepared in a manner similar to that set forth in Example 3:

| | |
|---|---:|
| ethoxylated bisphenol A dimethacrylate | 25.34 |
| benzoyl peroxide | 0.39 |
| tertiary amine | 0.275 |
| UV absorber | 0.115 |
| 2,6-di-tertiary-butyl-para-cresol | 0.025 |
| submicron silicon dioxide | 8.08 |
| strontium aluminium borosilicate | 31.025 |
| barium aluminium silicate | 34.75 |
| | 100.00 |

An abrasion rate of 1.09 microlitres/hour was measured for this material, seven days from the time of mixing.

These examples show not only the scope of the present invention, compared to previously-mentioned commercially-available materials, but also the effects of compositional variations on the abrasion rate of the respective materials.

Many compounds may be used as typical ingredients serving the functions of catalyst, accelerator and inhibitor and/or UV absorber components, such as:

CATALYSTS
dibenzoyl peroxide
lauryl peroxide
benzoyl acetyl peroxide
dicyclohexyl peroxide
ACCELERATORS
dimethyl-para-toluidine
diethyl-para-toluidine
dihydroxyethyl-para-toluidine
N,N-dimethyl-3,5-xylidine
para-(dimethylamino)-phenylacetic acid
INHIBITORS
hydroquinone monomethyl ether
2,6-di-tertiary-butyl-para-cresol
2-tertiary-butyl-4-methoxyphenol
3-tertiary-butyl-4-methoxyphenol

The catalysts, accelerators and inhibitors may typically be present in concentrations of up to 1.0 weight percent. Other additives, such as UV absorbers, are typically present in concentrations of up to 0.5 weight percent.

Very many different polymeric matrix and filler combinations have been investigated with respect to abrasion resistance, using a "protomatic" toothbrush abrasion machine, fitted with Colgate-Palmolive "medium" toothbrush heads, running in a simulated toothpaste slurry at 140 strokes per minute, with a fixed load of 220 grams and a stroke of 5.7 cm (2.25 inches) for a total of 67,200 strokes per specimen, over a swept area of 9.7 cm (1.5 square inches). The results obtained for six commercial composite restoratives and the best six materials (Examples 3 - 8) of the present invention were as follows:

TABLE

|  |  | Microlitres/ hour Abrasion Rate** |
|---|---|---|
| "Silar" * (Microfilled) | 3M Company | 19.80 |
| "Vytol" * | L.D.Caulk Co. | 3.33 |
| "Profile" * | S.S.White | 2.34 |
| "Simulate" * | Sybron/Kerr | 2.17 |
| "Concise" * | 3M Company | 2.15 |
| "Adaptic" *(Radiopaque) | J & J | 1.95 |
| Strontium glass/BISGMA/TEGDMA/BGDMA | | 1.81 |
| Strontium glass/PBPADMA | | 1.55 |
| Strontium glass/EBPADMA/BISGMA | | 1.46 |
| Strontium glass/EBPADMA | | 1.31 |
| Strontium glass/barium aluminosilicate/EBPADMA | | 1.12 |
| Strontium glass/barium aluminosilicate/EBPADMA | | 1.09 |

* Trademark of respective company listed in Table

** Measured seven days from time of mixing

It is readily seen that the abrasion resistance of commercial composites varies considerably and even the worst have been claimed to be satisfactory in clinical studies using anterior teeth and selected classes of cavity. Among the materials of the present invention, namely the last six materials listed in the Table, it can be seen that for a given filler system the abrasion resistance is always greater for the polymeric matrixes of this invention and that the commercially available radiopaque vitreous fillers utilized indicate that strontium-containing fillers generally exhibit superior abrasion resistance.

CLAIMS:

1. A dental restorative composition curable by means of a free-radical-generating peroxide/amine redox combination and comprising an acrylic functional monomer and at least one filler material, characterised in that the monomer comprises at least one alkoxylated bisphenol A dimethacrylate and that the filler material is selected from strontium aluminium borosilicate, barium aluminium silicate, barium aluminium borosilicate and mixtures thereof.

2. A dental restorative composition according to claim 1, wherein the monomer comprises ethoxylated bisphenol A dimethacrylate.

3. A dental restorative composition according to claim 1 or 2, wherein the monomer comprises propoxylated bisphenol A dimethacrylate.

4. A dental restorative composition according to claim 1, 2 or 3, wherein the monomer is either or both of ethoxylated bisphenol A dimethacrylate and propoxylated bisphenol A dimethacrylate blended with bisphenol A/glycidyl methacrylate adduct.

5. A dental restorative composition according to any preceding claim, which comprises 15-30 weight percent of ethoxylated bisphenol A dimethacrylate and/or propoxylated bisphenol A dimethacrylate blended with bisphenol A/glycidyl methacrylate adduct and 70-85 weight percent of the filler material.

6. A cured composite dental restoration composition comprising an acrylic functional monomer and at least one filler material, characterised in that the cured composition consists essentially of:

(a) 15-30 weight percent of at least one material selected from ethoxylated bisphenol A dimethacrylate and propoxylated bisphenol A dimethacrylate;

(b) up to 15 weight percent of bisphenol A/glycidyl methacrylate adduct;

(c) an activator;

(d) a catalyst;

(e) 70-85 weight percent of filler material selected from strontium aluminium borosilicate, barium aluminium silicate, barium aluminium borosilicate and mixtures thereof.

7. A cured two-paste filled composite restorative material based on the standard peroxide/ amine curing system and having the following final mix composition by weight:

| | |
|---|---|
| ethoxylated bisphenol A dimethacrylate ) | 15-30 combined |
| bisphenol A/glycidyl methacrylate adduct ) | |
| benzoyl peroxide | 0.1-1.0 |
| tertiary amine | 0.1-1.0 |
| UV absorber | 0.05-0.50 |
| 2,6-di-tertiary-butyl-para-cresol | 0.01-0.10 |
| strontium aluminium borosilicate ) | 70-85 combined |
| barium aluminium borosilicate ) | |
| TOTAL | 100.00 |

8. A cured two-paste composite material having the following final mix composition by weight:

| | |
|---|---|
| propoxylated bisphenol A dimethacrylate | 15-30 |
| benzoyl peroxide | 0.1-1.0 |
| UV absorber | 0.05-0.50 |
| 2,6-di-tertiary-butyl-para-cresol | 0.01-0.10 |
| submicron silicon dioxide | 1.0-10.0 |
| barium aluminium silicate | 70-85 |
| TOTAL | 100.00 |

9. A peroxide/amine cured two-paste composite material having the following final mix composition by weight:

| | | |
|---|---|---|
| ethoxylated bisphenol A dimethacrylate | | 15-30 |
| benzoyl peroxide | | 0.1-1.0 |
| tertiary amine | | 0.1-1.0 |
| UV absorber | | 0.05-0.50 |
| 2,6-di-tertiary-butyl-para-cresol | | 0.01-0.10 |
| submicron silicon dioxide | | 1.0-10.0 |
| barium aluminium silicate | ) | |
| strontium aluminium borosilicate | ) | 70-85 combined |
| | TOTAL | 100.00 |